Europäisches Patentamt

European Patent Office    (11) Publication number: **0 293 054**

Office européen des brevets    **A2**

## EUROPEAN PATENT APPLICATION

(21) Application number: 88201057.2    (51) Int. Cl.⁴: **C07D 303/27 , C08G 59/24**

(22) Date of filing: **25.05.88**

(30) Priority: **27.05.87 GB 8712454**

(43) Date of publication of application:
**30.11.88 Bulletin 88/48**

(84) Designated Contracting States:
**BE DE ES FR GB IT NL SE**

(71) Applicant: **SHELL INTERNATIONALE RESEARCH MAATSCHAPPIJ B.V.**
**Carel van Bylandtlaan 30**
**NL-2596 HR Den Haag(NL)**

(72) Inventor: **The other inventor has agreed to waive his entitlement to designation**

(74) Representative: **Aalbers, Onno et al**
**P.O. Box 302**
**NL-2501 CH The Hague(NL)**

(54) Novel diglycidyl ethers of diphenolic alkanes and their use in curable compositions.

⑤ Diglycidyl ethers of a bisphenolic alkane having from 5 to 14 carbon atoms in the alkane moiety, characterized in that the bisphenolic alkane is a reaction product of one mol of a linear α,ω-diolefin having from 5 to 14 carbon atoms per molecule, and two moles of a mono- or polycyclic monohydric phenol having at least a hydrogen atom in an ortho or in the para position in respect of the phenolic hydroxyl group.

EP 0 293 054 A2

# NOVEL DICLYCIDYL ETHERS OF DIPHENOLIC ALKANES AND THEIR USE IN CURABLE COMPOSITIONS

The invention relates to novel diglycidyl ethers of diphenolic alkanes, to their preparation and to their use in curable compositions, for example in combination with amino compounds for use in coatings, castings, adhesives and related applications.

Diglycidyl ethers of 2,2-bis(4-hydroxyphenyl)-propane (abbreviated DPP) of large variety in molecular weight are well-known commercial products. They are known as epoxy resins, and find use on a large scale as main resin component in thermosetting compositions.

For some purposes, however, certain properties of the epoxy resins from DPP need improvement e.g. the cured products derived therefrom show insufficient flexibility.

A method to provide glycidyl ethers of which the cured products may be expected to show improved flexibility has been described in GB-A 726,830. Said glycidyl ethers are derived from phenolic condensation products, which are the reaction product of a phenol having on its nucleus an unsaturated hydrocarbon substituent containing 14 to 28 carbon atoms, whose unsaturation is solely due to one or more ethylenic linkages, with a monohydric phenol derived from benzene, naphthalene or anthracene or their lower alkyl substituted homologues. The relatively large hydrocarbon moiety separating the phenolic nuclei of these phenolic condensation products together with the relatively high side chain content in said hydrocarbon moiety imparts a low hardness and a poor solvent resistance to thermoset products made by curing the aforesaid condensation products. Hence they cannot be applied in surface coatings.

The problem underlying the present invention is to provide glycidyl ethers which when used in coating composition result in flexible coatings having nevertheless an attractive hardness and solvent resistance.

It has now been found that this problem is solved by providing diglycidyl ethers derived from certain bisphenols which are reaction products of one mol of a linear $\alpha,\omega$-diolefin having from 5 to 14 carbon atoms and two moles of a mono- or polycyclic monohydric phenol having at least a hydrogen atom in an ortho or in the para position in respect of the phenolic hydroxyl group.

In the context of the present invention the term "diglycidyl ether" refers to glycidyl ethers having on average n epoxy groups per molecule and wherein $1 < n \leq 2$.

The monocyclic monohydric phenols which may be employed in the present invention may be phenol itself or a derivative thereof such as an alkyl, alicyclic, aryl, aralkyl or a halogen substituted phenol. Examples of such substituted phenol derivatives include cresols, xylenols, monohydroxy diphenyls, monochlorophenols, monobromophenols, dichlorophenols and dibromophenols. Phenol is a preferred monocyclic monohydric phenol. Examples of suitable polycyclic monohydric phenols include naphthols, anthrols and phenanthrols as well as the alkyl, alicyclic, aryl, aralkyl and halogen substituted derivatives thereof.

The linear $\alpha,\omega$-diolefin having from 5-14 carbon atoms per molecule comprise for example 1,4-pentadiene, 1,5-hexadiene, 1,6-heptadiene, 1,7-octadiene, 1,8-nonadiene, 1,9-decadiene, 1,10-undecadiene, 1,11-dodecadiene, 1,12-tridecadiene and 1,13-tetradecadiene.

The preparation of bisphenolic reaction products of $\alpha,\omega$-diolefins and monohydric phenols is known per se and may be conducted via a staged addition of the diolefin to a reactor containing a stirred mixture comprising a molar excess of monohydric phenol vis-à-vis the equivalents of olefinic unsaturation of the diolefin to be added and a desired amount of an acid catalyst at a temperature in the range of from 50 to 200 °C. Upon completion of the diolefin addition heating may be continued until the reaction has reached a sufficient degree of completion.

Subsequently, the catalyst is deactivated and the bisphenolic reaction product may be isolated. Although the nature of the acid catalyst, which may be employed in the preparation of said bisphenolic reaction product, is not critical, it has been found beneficial in the practice of the present invention to employ an acid clay type catalyst, such as Fulcat 22 B (Trade name). The use of such a catalyst in the process as described hereinbefore not only results in a high yield of bisphenolic reaction product, but also the catalyst can be separated from the reaction mixture by simple filtration.

Depending on the nature of the monohydric phenol employed, i.e. the number of hydrogen atoms in the para and/or in an ortho position to that of the phenolic hydroxyl group, the reaction product may be a single bisphenolic alkane or a mixture of isomers thereof. When the monohydric phenol has hydrogen atoms in both the para and the ortho positions the reaction product will generally be a mixture of the p,p′ - and o,p-isomers.

When the monohydric phenol itself is already a mixture of isomers as such, as is possible when the monohydric phenol is a substituted phenol as described hereinbefore, then this may also contribute towards the number of isomers present in the reaction product.

A further possible source for the occurrence of isomers in the reaction product may originate from isomerization within the $\alpha,\omega$-diolefin chain, which has sometimes been known to occur when such olefins are used in acid catalyzed processes as described hereinbefore.

At room temperature and slightly above, these bisphenolic alkanes or their isomeric mixtures are viscous to semi-solid, and can be diluted with a solvent or the reagent required for conversion into glycidyl ethers.

The bisphenolic alkanes or their isomeric mixtures, hereinafter referred to as bisphenols, can for example, be diluted easily with epichlorohydrin and/or alcohols, such as methanol, ethanol, n-propanol, isopropanol, n-butanol, isobutanol, and sec.-butanol.

The bisphenols can be converted into diglycidyl ethers by reaction with epichlorohydrin and alkali metal hydroxide, by the methods known for the preparation of glycidyl ethers of 2,2-bis(4-hydroxyphenyl)propane.

Liquid epoxy resins can be prepared by reacting the bisphenol of this invention with a molar excess of epichlorohydrin, if desired first in a pre-reaction in the presence of a catalyst-promoting formation of chlorohydrin ethers, whereafter the equivalent amount, or preferably an excess, of alkali metal hydroxide is added in one or more stages for dehydrohalogenation.

Higher molecular weight diglycidyl ethers can be prepared by reacting the bisphenol in aqueous medium with less than the equivalent amount of epichlorohydrin and with alkali metal hydroxide in one stage. Similar products can also be prepared by a fusion reaction of the liquid diglycidyl ether with a calculated amount (underdose) of the same bisphenol in the presence of a small amount of a catalyst, for example a tertiary amine, a quaternary ammonium salt, or a quaternary phosphonium salt, for example as described in GB-A 1,204,760 and GB-A 1,364,804. Other types of solid diglycidyl ethers can be prepared by a fusion reaction of liquid diglycidyl ethers according to the invention, in the presence of a catalyst as referred to above, with a different bisphenol, for example with 2,2,-bis-(4-hydroxyphenyl)propane.

The diglycidyl ethers according to the invention can be modified by reaction with all types of modifying agents known for the usual epoxy resins, for example with fatty acid to prepare epoxy resin esters, with amines (excess) to prepare soluble epoxy resin/amine adducts, with dicarboxylic acids (underdose) to prepare epoxy resins having a dicarboxylic acid ester group in the chain.

Diglycidyl ethers according to the invention can be converted to hard resinous materials by mixing and reacting with an epoxy resin curing agent. Useful curing agents are amino compounds having at least two amino hydrogen atoms per molecule. Examples are aliphatic polyamines, aromatic polyamines, soluble adducts thereof with mono-epoxides and/or di-epoxides, amino amides derived from aliphatic polyamines and carboxylic acids, heterocyclic polyamines. The cure with amino compounds may be accelerated by phenols or monocarboxylic acids. Other useful curing agents are polycarboxylic acids and polycarboxylic acid anhydrides; the cure may be accelerated by small amounts of catalysts, such as tertiary amines, for example benzyldimethylamine, or 2,4,6-tris-(dimethylaminomethyl)phenol, stannous salts, and others.

Solvents, diluents, extenders (coal tar, asphaltic bitumen), fillers, and pigments may also be added, depending on the intended use.

The invention will further illustrated by the following Examples.

## EXAMPLE I

### Preparation of a bisphenolic reaction product of 1,9-decadiene and phenol

Phenol (470 g, 5 moles) and a commercially available acid clay ("Fulcat 22 B" ex Laporte Industries Ltd., 17 g) were heated with stirring in a nitrogen atmosphere. 1,9-Decadiene (138 g, 1 mol) was added dropwise to the thus obtained mixture over a period of two hours, while maintaining the temperature at 115-120 °C. Upon completion of the diene addition heating and stirring were continued for three more hours whereupon the reaction mixture was cooled to 50 °C, diluted by the addition of methylene chloride (500 ml) and the acidic clay removed by filtration. The solvent and excess phenol were removed by distillation and steamstripping at 130 °C and under subatmospheric pressure to provide 250 g of the bisphenolic reaction product having a pale-brown colour and a phenolic hydroxyl content: 6 mol/kg.

## EXAMPLE II

### Diglycidyl ether preparation

The bisphenolic reaction product prepared in Example I (83.3 g, 500 mmol phenolic OH) was dissolved in a mixture of epichlorohydrin (277 g, 3 mol), 2-propanol (162 g) and water (40.5 g). The resulting mixture was heated with stirring to 40 °C, whereupon a 20 %w aqueous caustic solution (100 g, 0.5 mol NaOH) was added while the temperature was maintained at 40-42 °C for another 0.5 hour, after which the mixture was allowed to settle. After phase separation had occurred, the aqueous phase was removed and the organic phase heated to 60 °C whereupon further caustic solution (25 g, 0.125 mol) was added and the mixture was stirred at 60 °C for five minutes. Subsequently the mixture was allowed to settle and after phase separation had occurred the organic phase was washed consecutively with 100 ml volumes of water, 2 %w aqueous NaH₂PO₄ solution, and again water, while intermittently removing the aqueous phase formed. After removal of the final aqueous phase the volatile components were removed by evaporation at 120 °C and subatmospheric pressure (1 mbar).

The resulting diglycidyl ether (95 g) was a pale yellow liquid resin having an epoxy group content of 3.75 mol/kg and a viscosity of 8 Pa.s at 23 °C.

EXAMPLE III

Polyamine crosslinked, diglycidyl ether-based coatings

The diglycidyl ether prepared in Example II (53.3 g, 0.2 epoxy equivalents) was dissolved in a mixture of toluene (8 g) and benzyl alcohol (8 g). To this solution diethylene triamine (4.1 g, 0.04 mol) was added and the clear lacquer mixture obtained was stirred at 40 °C for one hour. Clear coatings having a dry film thickness of 40 μm were applied onto degreased steel panels by means of a bar coater and stoved at 130 °C for one hour. The resulting coatings were glossy and well-cured as can be seen from following film properties:
König hardness : 180 s
solvent resistance: > 50 toluene double-rubs
mandrel bend : 1/8 inch pass.

**Claims**

1. Diglycidyl ethers of a bisphenolic alkane having from 5 to 14 carbon atoms in the alkane moiety, characterized in that the bisphenolic alkane is a reaction product of one mol of a linear α,ω-diolefin having from 5 to 14 carbon atoms per molecule, and two moles of a mono- or polycyclic monohydric phenol having at least a hydrogen atom in an ortho or in the para position in respect of the phenolic hydroxyl group.

2. Diglycidyl ethers as claimed in claim 1, wherein the monocyclic phenol is phenol or an alkyl, alicyclic, aryl, aralkyl or halogen substituted phenol.

3. Diglycidyl ethers as claimed in claim 1, wherein the polycyclic phenol is napthol, anthrol, phenanthrol or an alkyl, alicyclic, aryl, aralkyl or halogen substituted naphtol, anthrol or phenantrol.

4. Diglycidyl ethers as claimed in any one of claims 1 to 3, wherein the bisphenolic alkane has been obtained by reacting in the presence of an acid clay type condensation catalyst.

5. Curable compositions and cured compositions based on a diglycidyl ether as claimed in any of claims 1 to 4.